# EUROPEAN PATENT SPECIFICATION

(11) **EP 2 905 013 B1**
(45) Date of publication and mention of the grant of the patent: **03.08.2016**
(21) Application number: 14154171.4
(22) Date of filing: 06.02.2014
(51) Int. Cl.: A61K 9/00, A61K 31/122

(54) **Treatment of East Coast Fever**
Behandlung von Ostküstenfieber
Traitement de la fièvre de la côte orientale

(43) Date of publication of application: 12.08.2015
(73) Proprietor: Bimeda AMEA Limited, Dublin 24 (IE)
(72) Inventor: McHardy, Nicholas, County Wicklow (IE)
(74) Representative: O'Brien, John Augustine

(56) References cited:
- MURAGURI ET AL: "Clinical efficacy and plasma concentrations of two formulations of buparvaquone in cattle infected with East Coast fever (Theileria parva infection)", RESEARCH IN VETERINARY SCIENCE, BRITISH VETERINARY ASSOCIATION, LONDON, GB, vol. 81, no. 1, August 2006 (2006-08), pages 119-126, XP005315869, ISSN: 0034-5288, DOI: 10.1016/J.RVSC.2005.09.012
- GANTALA VENKATESH ET AL: "In vitro and in vivo evaluation of self-microemulsifying drug delivery system of buparvaquone", DRUG DEVELOPMENT AND INDUSTRIAL PHARMACY, vol. 36, no. 6, June 2010 (2010-06), pages 735-745, XP055123104, ISSN: 0363-9045, DOI: 10.3109/03639040903460446
- MANTYLA A ET AL: "Design, synthesis and in vitro evaluation of novel water-soluble prodrugs of buparvaquone", EUROPEAN JOURNAL OF PHARMACEUTICAL SCIENCES, ELSEVIER, AMSTERDAM, NL, vol. 23, no. 2, October 2004 (2004-10), pages 151-158, XP004580038, ISSN: 0928-0987, DOI: 10.1016/J.EJPS.2004.06.006

## Description

### Introduction

East Coast Fever (ECF) is a form of theileriosis caused by the parasite *Theilerice parva* transmitted by the tick *Rhipicephalus appendiculatus.* It is a major disease of cattle in at least 11 countries in eastern, central and southern Africa. The affected countries include Burundi, Kenya, Malawi, Mozambique, Rwanda, South Sudan, Tanzania, Uganda, Zaire, Zambia and Zimbabwe. Tropical theileriosis (TT) of cattle, caused by *Theileria annulata* and transmitted by several species of ticks, is equally severe. Affected countries include Morocco, Tunisia, Egypt, Sudan, Syria, Iraq, Iran, Pakistan, India and several other countries in Southern Europe, Southern Asia, Middle East and North Africa.

Conventional treatments for ECF and TT are solutions for intramuscular injection. Hypodermic syringes and needles are required and a veterinarian is generally required to administer them. Conventional drugs must be placed intramuscularly. They are far less effective if placed subcutaneously, and they may cause severe and potentially fatal shock if misplaced intravenously. In addition, particularly in small calves, there is a danger that the injecting needle may strike a bone and this can cause severe adverse side effects.

Another problem is that injections are presented in glass vials, which can be broken accidentally, and such liquid products are generally unstable at high temperatures such as are found in direct sunlight or in unrefrigerated storage or transportation vehicles and containers in eastern, central and southern and northern Africa, southern Europe, Middle East and southern Asia.

Muraguri et al, "Clinical efficacy and plasma concentrations of two formulations of buparvaquone in cattle infected with East Coast fever (Theileria parvainfection)", Research in Veterinary Scieuce, 2006; 81: 119-126, discloses intramuscular formulations of buparvaquone for the treatment of East Coast fever.

### Statements of Invention

According to the invention there is provided an intra-ruminal bolus for oral administration to a ruminant animal comprising buparvaquone in a bolus formulation.

In one embodiment the bolus comprises from 50 to 250 mg of buparvaquone. The bolus may comprise approximately 100 mg of buparvaquone.

In one embodiment the bolus is dividable. The bolus may comprise at least one groove for ease of division of the bolus.

The invention also provides a bolus comprising buparvaquone in an amount to deliver approximately 5 mg/Kg of the bodyweight of a ruminant.

The ruminant animal may be a bovine animal such as a calf.

The bolus of the invention is useful for the treatment of East Coast Fever and/or Tropical theileriosis.

It is surprising that the oral dose bolus is effective in the treatment of ECF or TT. Buparvaquone (BPQ) is almost entirely insoluble in water. For a drug to be absorbed from the gut (which is necessary for it to act against the ECF or TT parasites which are located within lymphoid, certain other white, and red blood cells), it must first become a solution, or an emulsion. The bolus formulation however contains nothing that would be expected to increase the solubility or emulsifiability of buparvaquone.

There are advantages of the bolus of the invention over all existing treatments for ECF and TT, all of which are solutions for intramuscular injection. These advantages apply particularly to calves, including very young calves. Administration of a bolus by mouth is relatively easy, so it can be done by calf owners without the expense of a veterinarian or other animal health professional. The bolus is simply placed over the back of the tongue, the mouth is held closed and the throat is stroked until the calf swallows the bolus.

In one case the bolus contains 100 mg of buparvaquone (BPQ) - enough to treat a 20 Kg calf. For larger calves the dose can be a multiple of boluses (or half boluses - the bolus is scored to make it easily broken in half), depending on the calf's weight. Calf owners know the approximate weight of their calves, so calculating the correct dose is easy. It is far easier than calculating the necessary dose of an injectable product, drawing it into the syringe and delivering it, without leakage or misplacement, by intramuscular injection.

### Detailed Description

The invention will be more clearly understood from the following examples.

### Example 1 - Bolus Formulation

| **Ingredient** | | **Amount in mg/bolus** | **Amount in Kg/batch** | **Function** |
|---|---|---|---|---|
| **Approved Name** | **Reference** | | | |
| Buparvaquone (BPQ) | - | 100 mg | 0.500 | Active |
| Microcrystalline Cellulose | B.P. | 434 mg | 2.170 | Diluent |
| Sodium Starch Glycolate | B.P. | 100 mg | 0.500 | Disintegrant |
| Lactose | B.P. | 1136 mg | 5.680 | Diluent |
| PVPK-30 | B.P. | 30 mg | 0.150 | Binder |
| Isopropyl Alcohol | B.P. | - | Q.S. | Vehicle |
| Purified Talcum | B.P. | 40 mg | 0.200 | Lubricant |
| Magnesium Stearate | B.P. | 20 mg | 0.100 | Lubricant |
| Cross Carmellose Sodium | B.P. | 115 mg | 0.575 | Disintegrant |
| Aerosil-200 | USP | 25 mg | 0.125 | Glidant |

A batch of 5,000 boluses was prepared by mixing, drying and blending the ingredients listed above. The mixture was formed into caplets using a tablet pressing technique. The uncoated caplets thus formed were light yellow coloured and elongated bioconvex in shape.

### Bolus

| | |
|---|---|
| Weight | 2gm |
| Thickness | 6.3mm |
| Diameter | 28mm |
| Hardness | 3.0 kg/cm³ |

The disintegration time of the bolus in a medium simulating *in vivo* use was approximately 2 minutes.

In the bolus of the invention the ingredients are present in the form of compressed powders or granules. The bolus may be prepared by direct compression of the admixed ingredients. When the core is prepared by granulation it may be convenient to add a lubricant such as magnesium stearate after the granulation step. The degree of compression will affect the hardness of the bolus.

### Example 2

A trial was conducted to demonstrate the efficacy and safety of the dividable bolus of Example 1 for oral delivery, containing 100mg of buparvaquone, and to indicate a likely dosage rate for the product under field conditions, for an effective and convenient product for the treatment of East Coast fever (ECF, *Theileria parva* infection) in calves, and by implication, also for the treatment of TT, *Theileria annulata* infection in calves.

A pilot study had demonstrated that approximately 50% of buparvaquone is absorbed into the blood after oral delivery. Therefore, if a dose of 5.0mg buparvaquone per Kg bodyweight is delivered orally, approximately 2.5mg buparvaquone /Kg bodyweight would be absorbed and become available to act against the theileriosis (ECF / TT) infection in the body. It is generally known, and extensively published, that a dose of 2.5mg buparvaquone/Kg bodyweight delivered by intramuscular injection is effective for the treatment of ECF and TT.

The test animals were high grade calves from a farm with no recent history of ECF. They were held in quarantine at the National Veterinary Research Centre (NVRC), Muguga, Kenya, for at least 14 days, to allow them to acclimatise and to ensure that they were free from disease and carrying no antibody to ECF. Antibody to ECF would indicate that they had experienced the disease in the past, and that they would be resistant to re-infection. They were kept housed and fed an appropriate high quality diet, including commercially available artificial milk substitute as appropriate.

The calves were artificially infected with a dose of the Marikebuni stock of ECF that has been shown, over many years, to cause a consistent pattern of disease and to kill more than 90% of calves so infected in about 14-21 days after infection.

Bolus treatments began on day 'TS3', i.e. the third day on which each calf had a rectal temperature (T) of 39.5°C or more, and schizonts (S) were present in smears taken from the prescapular lymph nodes. This would be expected to be around 12-14 days after infection, when the ECF would be relatively severe.

Three groups of calves were included in the study:

| | |
|---|---|
| Group 1. | Untreated controls |
| Group 2. | 1 BPQ bolus / 20Kg bodyweight, daily, until cured |
| Group 3. | 1 BPQ bolus / 20Kg bodyweight on alternate days until cured |

The boluses are dividable, so the bolus dose was rounded to the nearest 10Kg bodyweight. Thus, a calf weighing 56-65Kg received 3 boluses, while a calf weighing 66-75Kg received 3.5 boluses. The buparvaquone dose, therefore, was a nominal 5mg/Kg per treatment.

"Cure" is defined as the second consecutive day on which temperature remains below 39.5°C and theilerial schizonts are either absent from, or are clearly dying, in prescapular lymph node smears. This being a pilot study designed to indicate a likely effective dose rate for use in a more extensive field study in naturally-infected calves, it was terminated soon after the untreated calves had died, and the bolus-treated calves had either died or been cured, of their ECF infections.

### MATERIALS AND METHODS

### Experimental animals

Fourteen bull calves aged 3-6 months and weighing 57-121 Kg, of pure or almost pure *Bos taurus* type were included in the study. Before purchase, smears from a prescapular lymph node were stained and examined microscopically for the presence of *Theileria* schizonts. Stained blood smears were examined for the presence of any other tick-borne infections and heparinised blood plasma was assayed for antibody to *Theileria* in the indirect fluorescent antibody test (IFAT). All results were negative. A thorough clinical examination showed that they were all in good health. They were transported by road to NVRC, Muguga.

On arrival at NVRC, a unique numbered ear-tag was attached to each calf, they were hand-sprayed with an amitraz-based acaricide and all of the above tests were repeated. They were repeated again two weeks later. All tests for infections were negative and all the calves were in good health on both occasions.

### Animal husbandry

The calves were housed on dry bedding in a closed tick-proof and rodent-free building with good ventilation. They were fed on good quality hay, commercially available milk replacement diet and concentrates, appropriate to their age.

### Infection of the calves with Theileria parva (ECF)

The calves were infected, in compliance with the appropriate NVRC Standard Operating Procedure (SOP), by subcutaneous injection above the left prescapular lymph node with 1.0ml of a 1 in 10 dilution of the NVRC standard stabilate of the Marikebuni stock. This infective dose has been shown, over many years, to result consistently in fatal ECF in more than 90% of susceptible cattle, usually within 14-21 days of infection. The day of infection was designated 'day 0' of the study.

### Observations

**Clinical:** All calves were examined clinically by a competent veterinarian for signs of ECF, any other tick-borne disease, or any other disease or condition, infective or otherwise, daily before 10.00 am. Rectal temperature was taken daily as an integral element of this examination.
**Lymph node smears:** Smears were prepared daily from the left prescapular lymph node (LPN) of all calves from day 7 and also from the right prescapular node (RPN) from day 10, in accordance with the NVRC SOP. *Theileria* schizont "score" was recorded:-
   0 = no schizonts found after careful examination
   1 = schizonts present, but difficult to find
   2 = schizonts easy to find
   3 = heavy schizont infection
**Blood smears:** Smears of ear-vein blood were prepared daily beginning two days after *Theileria* schizonts were first detected in lymph node smears. *Theileria* piroplasm parasitaemia was scored as percent of erythrocytes containing a piroplasm. Blood smears were also examined for the presence of other intra-erythrocytic parasites, i.e. *Babesia bigemina* and *Anaplasma marginale.*

Lymph node and blood smears were fixed with methanol and stained with Giemsa's stain, then examined microscopically on the day that they were prepared, in accordance with the appropriate NVRC SOP. All microscope slides were identified by animal number, date of sampling and type of smear (e.g. LPN, RPN, B), but not the treatment group of the calf, so as to maintain "blinding" during microscopical examination.

### Medication

**Test substance and dosage:** The substance under test was the antitheilerial compound buparvaquone (BPQ) formulated as a pale yellow dividable bolus for oral administration. Each bolus contained 100mg BPQ. Dosage rate was one bolus per 20 Kg bodyweight, equivalent to a nominal 5mg BPQ per Kg:-

| | |
|---|---|
| 56 - 65 Kg | 3 boluses |
| 66 - 75 Kg | 3.5 boluses |
| 76 - 85 Kg | 4 boluses |
| 86 - 95 Kg | 4.5 boluses |
| etc. | |

Boluses were administered to the restrained calf by placement on the back of the tongue, holding the mouth closed and gently massaging the throat until the bolus was swallowed. The buccal cavity was then examined to confirm that the bolus had been swallowed.
**Other medication:** All calves were treated with acaricide weekly from the day of arrival at NVRC. They were treated with an anthelmintic on the day of arrival only. The protocol allowed for additional medicine to be administered for infections and conditions other than ECF as may be necessary (except that tetracycline antibiotics should not be used as they might have a marginal effect on theileriosis).

### Study design

The calves were assigned to the three groups strictly in the order in which they attained "TS3" status - i.e. three days of temperature of 39.5°C or higher with *Theileria* schizonts present in lymph node smears. The groups were:

| | | |
|---|---|---|
| **Group 1** | **U ntreated controls** | **3 calves** |
| **Group 2** | **T reated daily from TS3 until cured** | **6 calves** |
| **Group 3** | **T reated on alternate days from TS3 until cured** | **5 calves** |

They were assigned to the groups in the sequence :- Gp.1, Gp.2, Gp.3, Gp. 2, Gp. 3, Gp. 1, Gp. 2, Gp. 3, Gp 2 ...... etc.

Bolus treatments were given at the same time each day to maintain 24- or 48-hourly intervals as closely as possible.

"Clinical cure" is defined as occurring when rectal temperature has remained below 39.5°C for two consecutive days and schizonts are absent from lymph node smears, or present in very small numbers but clearly damaged by the treatment.

The study was ended as soon as all calves had either died of ECF or had been cured of their infection.

### Post mortem examinations

A detailed *post mortem* examination was conducted on all animals that died during the study.

### Record keeping

All procedures, observations and medications on-farm before purchase of the calves, and during quarantine at NVRC were recorded in laboratory notebooks.

From the start of the study, "Day 0", the day of infection with *Theileria* stabilate, all procedures, observations - clinical, parasitological and pathological, *post mortem* findings and all medications, were recorded on individual Animal Record Sheets and / or in laboratory notebooks.

### RESULTS

All 14 calves developed clinical ECF. They attained "TS3" status in 11-13 days after infection.

All three untreated control calves died, on days 14, 16 and 17 respectively. The cause of death in all three cases was ECF.

All six calves treated daily with buparvaquone boluses were cured. Temperature was below 39.5°C in five of them on the day after first treatment, and in the sixth on the second day. All temperatures then remained below 39.5°C until the end of the study, eight days after first treatment. Schizont parasitosis was reduced in all six calves by the third day after first treatment, then it fluctuated at low / negative levels until the end of the study. Clear signs of damage to the schizonts, typical of the effects of buparvaquone, were seen in the smears of all of the calves by the third day after first bolus treatment. Small numbers of *Theileria* piroplasms were observed in the blood smears of some of the calves at the time of first treatment, but smears were all negative for piroplasms within two days of first treatment.

Four of the five calves treated with buparvaquone boluses on alternate days were cured, but one died of ECF on the fifth day after first treatment. It had appeared to be recovering by the second day after first bolus treatment, but its temperature was sub-normal (36.0°C) from the third day, when clear signs of advanced clinical ECF became apparent. The four calves in this group that survived showed a pattern of recovery essentially similar to that of the calves that were treated daily.

No signs of any adverse effects that might be associated with the administration of the buparvaquone treatments were seen.

The temperatures and schizont scores of all the calves are shown in Tables 1 and 2.

No *Babesia* or *Anaplasma* parasites were seen in blood smears at any time.

In view of the persistent, although very low and sporadic, schizont parasitosis seen among the treated calves following clinical recovery from ECF, and because of the early death of one of them, bolus treatment was continued in all of them until the end of the study (day TS+8), as a precaution for their welfare.

### ANALYSIS AND CONCLUSIONS

1. The course of the ECF seen in the calves in this study was rapid and severe, as shown by the relatively short time to achieve "TS3", 11-13 days, and the relatively early death of the untreated controls, in 14-17 days. The low piroplasm parasitaemias and minimal weight changes, even in the untreated calves, also suggest strongly that the ECF in this study was acute. The challenge to the treatments, therefore, was probably at least as severe as is likely to be encountered among naturally infected calves, on farms.
2. Daily treatment with boluses delivering a nominal 5mg buparvaquone / Kg bodyweight at each treatment was clearly effective in controlling fulminating and severe ECF, since all six calves receiving this treatment were quickly cured and bodyweight was maintained. The response was similar to that seen following treatment with an injectable preparation of buparvaquone at a dose rate of 2.5mg/Kg bodyweight, such as Buparvex (Bimeda).
3. Treatment at the same dose rate on alternate days cured four of five calves, but one died of ECF. However, the other four responded essentially similarly to those receiving daily treatment. The calf that died was moribund at the time that it received its second bolus treatment, having attained "TS3" only 12 days after infection, the same as in the first untreated control to die. It seems likely, therefore, that the ECF in this calf was among the most rapidly fulminating in the study.
4. In retrospect, it seems likely that continuation of treatments until the end of the study because of the persistence of small numbers of mostly drug-damaged schizonts was overly cautious. The calves treated daily received seven treatments, those treated on alternate days received four. Except for the one treated calf that died, it seems probable that the cure rate would not have been reduced had fewer treatments been given.
5. None of the calves showed any adverse effects that might be attributed to treatment with the buparvaquone boluses. The calves treated daily received a total of 35mg buparvaquone / Kg bodyweight. The lack of adverse effects is unsurprising given the known extremely low toxicity of BPQ. It shows also that the excipients of the bolus are safe, even with these frequent doses.
6. The study indicates that daily treatment of naturally occurring ECF in calves, with BPQ boluses at a nominal dose rate of 5mg buparvaquone / Kg bodyweight, would be effective, safe and a convenient method of treatment, and it would achieve cure rates comparable with those currently achieved with injectable preparations of buparvaquone. Given that death within 14 days of infection with ECF under field conditions is unusual, it seems likely that treatment with buparvaquone boluses on alternate days could also be effective.
7. Livestock owners would be likely to recognise the signs of ECF, and begin treatment, earlier than "TS3" in most cases. This gives added assurance that the buparvaquone bolus dosages suggested in (6) would be effective under field conditions.

**Table 1. Rectal temperature, °C. Treatment began on day TS3.**

| **Day :** | **-6** | **-5** | **-4** | **-3** | **-2** | **-1** | **TS3** | **+1** | **+2** | **+3** | **+4** | +**5** | **+6** | **+8** |
|---|---|---|---|---|---|---|---|---|---|---|---|---|---|---|
| | | | | | | | | | | | | | | |
| **Untreated controls** | | | | | | | | | | | | | | |
| **094L** | 38.6 | 38.6 | 39.1 | 38.8 | 39.9 | 39.8 | 39.9 | 39.7 | 36.0 | **D** | | | | |
| **107L** | 37.0 | 37.8 | 37.5 | 38.1 | 40.1 | 40.3 | 39.7 | 40.3 | 39.9 | 39.5 | 36.5 | **D** | | |
| **115L** | 38.5 | 38.7 | 38.4 | 38.6 | 40.1 | 40.5 | 41.1 | 41.6 | 41.3 | **D** | | | | |
| **Mean** | **38.4** | **38.4** | **38.3** | **38.5** | **40.0** | **40.2** | **40.2** | **40.5** | **40.6** | | | | | |
| | | | | | | | | | | | | | | |
| **Treated daily from TS3 with BPQ boluses** | | | | | | | | | | | | | | |
| **096L** | 38.8 | 39.6 | 39.0 | 39.7 | 39.7 | 39.8 | 40.4 | 39.0 | 38.1 | 39.6 | 38.7 | 38.1 | 38.7 | 38.0 |
| **108L** | 38.4 | 39.2 | 39.9 | 39.9 | 39.6 | 39.7 | 39.1 | 39.0 | 38.4 | 38.3 | 37.2 | 38.3 | 38.0 | 38.3 |
| **110L** | 37.6 | 38.4 | 38.0 | 39.6 | 38.5 | 40.3 | 41.1 | 40.8 | 38.6 | 38.5 | 39.4 | 38.0 | 39.7 | 39.1 |
| **117L** | 38.4 | 38.3 | 38.6 | 39.2 | 39.5 | 40.1 | 40.4 | 39.2 | 38.5 | 38.7 | 38.6 | 38.1 | 38.7 | 38.7 |
| **118L** | 38.3 | 37.6 | 38.6 | 38.5 | 39.7 | 39.7 | 39.6 | 39.2 | 38.4 | 38.2 | 38.3 | 38.0 | 38.0 | 39.0 |
| **119L** | 38.3 | 38.6 | 38.7 | 39.3 | 39.5 | 39.9 | 40.1 | 39.4 | 38.4 | 38.5 | 38.4 | 38.2 | 38.5 | 38.4 |
| **Mean** | **38.3** | **38.6** | **38.7** | **39.3** | **39.5** | **39.9** | **40.1** | **39.4** | **38.4** | **38.5** | **38.4** | **38.2** | **38.5** | **38.4** |
| | | | | | | | | | | | | | | |
| **Treated on alternate days from TS3 with BPQ boluses** | | | | | | | | | | | | | | |
| **102L** | 38.1 | 38.8 | 39.5 | 38.7 | 39.5 | 39.7 | 39.6 | 38.6 | 38.3 | 38.7 | 39.1 | 38.5 | 38.9 | 38.0 |
| **111L** | 38.3 | 37.7 | 38.6 | 39.2 | 39.9 | 39.7 | 40.0 | 39.7 | 39.7 | 38.3 | 38.7 | 38.6 | 39.1 | 38.1 |
| **113L** | 37.8 | 37.9 | 38.1 | 38.6 | 39.6 | 39.4 | 40.1 | 39.3 | 41.1 | 40.8 | 38.4 | 37.6 | 38.5 | 38.7 |
| **114L** | 37.6 | 38.4 | 38.6 | 39.3 | 40.0 | 40.4 | 39.4 | 39.5 | 38.5 | 38.7 | 38.1 | 38.5 | 37.8 | 37.8 |
| **116L** | 37.0 | 39.2 | 38.5 | 39.0 | 40.1 | 39.4 | 39.8 | 38.9 | 38.0 | 36.0 | 35.2 | **D** | | |
| **Mean** | **37.8** | **38.4** | **38.7** | **39.0** | **39.8** | **39.7** | **39.8** | **39.2** | **39.1** | **39.1** | **38.6** | **38.3** | **38.6** | **38.1** |

**Table 2. Schizont scores, maximum 6.0**

| **Day** : | **-6** | **-5** | **-4** | **-3** | **-2** | **-1** | **TS3** | +**1** | +**2** | **+3** | +**4** | +**5** | **+6** | **+8** |
|---|---|---|---|---|---|---|---|---|---|---|---|---|---|---|
| | | | | | | | | | | | | | | |
| **Untreated controls** | | | | | | | | | | | | | | |
| **094L** | | 0 | 0 | 0 | 0 | 1 | 1 | 2 | 2 | **D** | | | | |
| **107L** | | 0 | 0 | 0 | 0 | 1 | 1 | 2 | 2 | 3 | 3 | **D** | | |
| **115L** | | 0 | 0 | 1 | 2 | 1 | 1 | 3 | 6 | **D** | | | | |
| **Mean** | | **0** | **0** | **0.3** | **0.6** | **1.0** | **1.0** | **2.3** | **3.3** | | | | | |
| | | | | | | | | | | | | | | |
| **Treated daily from TS3 with BPQ boluses** | | | | | | | | | | | | | | |
| **096L** | | 0 | 0 | 0 | 0 | 0 | 1 | 1 | 0.5 | 0 | 0.5 | 0 | 0 | 0 |
| **108L** | | 0 | 0 | 0 | 0 | 0 | 1 | 0.5 | 0 | 0.5 | 0.5 | 0 | 0 | 0 |
| **110L** | | 0 | 0 | 0 | 1 | 2 | 3 | 3 | 0.5 | 1 | 3 | 0 | 0 | 0 |
| **117L** | | 0 | 0 | 0 | 1 | 1 | 2 | 1 | 1 | 1 | 0.5 | 0.5 | 0 | 0 |
| **118L** | | 0 | 0 | 0 | 1 | 1 | 2 | 1 | 1 | 1 | 0.5 | 0.5 | 1 | 0 |
| **119L** | | 0 | 0 | 0 | 1 | 1 | 1 | 1 | 1 | 0.5 | 0 | 0.5 | 0 | 0.5 |
| **Mean** | | **0** | **0** | **0** | **0.7** | **0.8** | **1.7** | **1.3** | **0.7** | **0.7** | **0.8** | **0.5** | **0.1** | **0.1** |
| | | | | | | | | | | | | | | |
| **Treated on alternate days from TS3 with BPQ boluses** | | | | | | | | | | | | | | |
| **102L** | | 0 | 0 | 0 | 0 | 1 | 2 | 2 | 2 | 0.5 | 0.5 | 1 | 0 | 0.5 |
| **111L** | | 0 | 0 | 0 | 1 | 1 | 2 | 2 | 1 | 0.5 | 0.5 | 0.5 | 0 | 0.5 |
| **113L** | | 0 | 0 | 0 | 0 | 1 | 1 | 0 | 0 | 0 | 0 | 0.5 | 0 | 0 |
| **114L** | | 0 | 0 | 0 | 1 | 1 | 1 | 1 | 1 | 0 | 0.5 | 0 | 0.5 | 0 |
| **116L** | | 0 | 0 | 0 | 2 | 2 | 2 | 1 | 2 | 0.5 | 0.5 | **D** | | |
| **Mean** | | **0** | **0** | **0** | **0.8** | **1.2** | **1.6** | **1.2** | **1.2** | **0.3** | **0.4** | **0.5** | **0.2** | **0.3** |

| | | | | | | | | | | | | | | |
|---|---|---|---|---|---|---|---|---|---|---|---|---|---|---|
| **Note : Score 0.5 indicates small number of drug-damaged or dead schizonts, of little or no pathological importance.** | | | | | | | | | | | | | | |

**Table 3. Bodyweight, Kg.**

| **Day** | **Infn.** | **TS3** | **TS3 + 8** | | **No. boluses / treatment** | |
|---|---|---|---|---|---|---|
| | | | | | | |
| **094L** | 87 | 83 | **D** | | 0 | |
| **107L** | 77 | 79 | **D** | | 0 | |
| **115L** | 73 | 75 | **D** | | 0 | |
| **Mean** | **79.0** | **79.0** | | | | |
| | | | | | | |
| **096L** | 65 | 69 | 69 | | 3.5 | |
| **108L** | 89 | 88 | 87 | | 4.5 | |
| **110L** | 79 | 75 | 77 | | 4.0 | |
| **117L** | 115 | 118 | 118 | | 6.0 | |
| **118L** | 115 | 118 | 115 | | 6.0 | |
| **119L** | 57 | 59 | 61 | | 3.0 | |
| **Mean** | **86.7** | **87.8** | **87.8** | | **4.5** | |
| | | | | | | |
| **102L** | 81 | 75 | 87 | | 4.5 | |
| **111L** | 65 | 67 | 67 | | 3.5 | |
| **113L** | 121 | 124 | 121 | | 6.0 | |
| **114L** | 77 | 79 | 81 | | 4.0 | |
| **116L** | 83 | 77 | **D** | | 4.0 | |
| **Mean** | **85.4** | **84.4** | **(89.0)** | | **4.4** | |

Cattle are ruminants. Ingested food first goes to the rumen, where it is fermented (among other things) but no absorption of nutrients occurs from the rumen. Absorption of nutrients begins only when the food enters the intestine. Drugs are absorbed only when they reach the intestine, which is several hours after they are ingested. Oral delivery of drugs to ruminants, therefore, entails a delay of several hours between administering the drug and its first being absorbed. However, the rumen does not become functional in calves until they are aged about six weeks. In calves aged less than about six weeks, the food passes directly into the intestine, so absorption of nutrients can begin much sooner than in older calves, and with an acute disease like ECF, this may be a significant advantage. It may also result in more complete absorption of the buparvaquone. The advantage may be two-fold - more rapid onset of effect and lower required drug dose.

The invention is not limited to the embodiment hereinbefore described, which may be varied in detail.

## Claims

1. An intra-ruminal bolus for oral administration to a ruminant animal comprising buparvaquone in a bolus formulation.

2. A bolus as claimed in claim 1 wherein the bolus comprises from 50 to 250 mg of buparvaquone.

3. A bolus as claimed in claim 1 wherein the bolus comprises approximately 100 mg of buparvaquone.

4. A bolus as claimed in any of claims 1 to 3 wherein the bolus is dividable.

5. A bolus as claimed in claim 4 wherein the bolus comprises at least one groove for ease of division of the bolus.

6. A bolus as claimed in any of claims 1 to 5 comprising buparvaquone in an amount to deliver approximately 5 mg/Kg of the bodyweight of a ruminant.

7. A bolus as claimed in any of claims 1 to 6 wherein the ruminant animal is a bovine animal.

8. A bolus as claimed in claim 7 wherein the bovine animal is a calf.

9. A bolus as claimed in any of claims 1 to 8 for use in the treatment of East Coast Fever.

10. A bolus as claimed in any of claims 1 to 8 for use in the treatment of Tropical theileriosis.

## Patentansprüche

1. Intraruminaler Bolus für die orale Verabreichung an ein wiederkäuendes Tier, der Buparvaquon in einer Bolus-Formulierung umfasst.

2. Bolus nach Anspruch 1, wobei der Bolus von 50 bis 250 mg Buparvaquon umfasst.

3. Bolus nach Anspruch 1, wobei der Bolus ungefähr 100 mg Buparvaquon umfasst.

4. Bolus nach einem der Ansprüche 1 bis 3, wobei der Bolus teilbar ist.

5. Bolus nach Anspruch 4, wobei der Bolus mindestens eine Rille zur Vereinfachung der Teilung des Bolus umfasst.

6. Bolus nach einem der Ansprüche 1 bis 5, der Buparvaquon in einer Menge umfasst, um ungefähr 5 mg/kg des Körpergewichts eines Wiederkäuers abzugeben.

7. Bolus nach einem der Ansprüche 1 bis 6, wobei das wiederkäuende Tier ein Rind ist.

8. Bolus nach Anspruch 7, wobei das Rind ein Kalb ist.

9. Bolus nach einem der Ansprüche 1 bis 8 für die Verwendung bei der Behandlung von Ostküstenfieber.

10. Bolus nach einem der Ansprüche 1 bis 8 für die Verwendung bei der Behandlung von tropischer Theileriose.

## Revendications

1. Bolus intra-ruminal pour une administration orale à un animal ruminant, comprenant de la buparvaquone dans une formulation de bolus.

2. Bolus selon la revendication 1, où le bolus comprend de 50 à 250 mg de buparvaquone.

3. Bolus selon la revendication 1, où le bolus comprend environ 100 mg de buparvaquone.

4. Bolus selon l'une quelconque des revendications 1 à 3, où le bolus est divisible.

5. Bolus selon la revendication 4, où le bolus comprend au moins un sillon pour faciliter la division du bolus.

6. Bolus selon l'une quelconque des revendications 1 à 5, comprenant de la buparvaquone selon une quantité pour administrer environ 5 mg/kg de poids corporel d'un ruminant.

7. Bolus selon l'une quelconque des revendications 1 à 6, où l'animal ruminant est un animal bovin.

8. Bolus selon la revendication 7, où l'animal bovin est un veau.

9. Bolus selon l'une quelconque des revendications 1 à 8, pour une utilisation dans le traitement de la Fièvre de la Côte Orientale.

10. Bolus selon l'une quelconque des revendications 1 à 8, pour une utilisation dans le traitement de la theilériose tropicale.
